(19)

Europäisches
Patentamt
European
Patent Office
Office européen
des brevets

(11) **EP 3 861 924 A1**

(12) **EUROPEAN PATENT APPLICATION**

(43) Date of publication:
**11.08.2021 Bulletin 2021/32**

(51) Int Cl.:
**A61B 3/16** (2006.01)  **A61F 9/00** (2006.01)

(21) Application number: **21154672.6**

(22) Date of filing: **02.02.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.02.2020  IT 202000002398**

(71) Applicant: **Alfa Intes Industria Terapeutica Splendore S.r.l.**
**80026 Casoria (NA) (IT)**

(72) Inventor: **DAVID, Alessio**
**I-80026 Casoria (NA) (IT)**

(74) Representative: **Porta & Consulenti Associati S.p.A.**
**Via Vittoria Colonna, 4**
**20149 Milano (IT)**

(54) **CONTACT LENS DEVICE AND RELATED PRESSURE MONITORING KIT AND SYSTEM COMPRISING THE SAME**

(57)    A contact lens device (10) comprising: a contact lens (11) extending radially about a central axis (Z) and comprising a central optical zone (26) and a radially outer zone (28) located concentrically around the central optical zone (26); an intraocular pressure sensor (14) joined to the contact lens (11), the pressure sensor (10) comprising a sensing layer (21) made of transparent dielectric material, a lower electrode (16) and an upper electrode (18), the sensing layer (21) being placed between the lower electrode (16) and the upper electrode (18) with respect to the central axis (Z), wherein the pressure sensor (14) is arranged on the central optical zone (26) of the contact lens (11), the contact lens (11) comprises a drug-releasing material (24) containing a drug, the drug-releasing material (24) being incorporated in the contact lens (11) within the radially outer zone (28) so as to be arranged peripherally the pressure sensor (14). A kit comprising the contact lens device (10) and a monitoring system (50) for wirelessly monitoring intraocular pressure are also described.

FIG. 1

FIG. 2

EP 3 861 924 A1

**Description**

Related field

The present disclosure relates to a contact lens device for monitoring intraocular pressure and drug delivery to the eye. The present disclosure is directed also to a kit and an intraocular pressure monitoring system comprising such a contact lens device.

Background

Glaucoma generally refers to a group of chronic and initially asymptomatic progressive neuropathies leading to early irreversible nerve fibres damage which can result in an impaired vision or a total blindness if inadequately treated. Because of the relatively asymptomatic nature of glaucoma, especially at an early stage, most individuals with glaucoma remain undiagnosed.

It is therefore important to have a screening to detect glaucoma and treat patients in an early stage to reduce the risk of visual loss. Screening of glaucoma is typically performed as part of an ocular examination carried out by ophthalmologists and clinicians and may include several eye tests or examinations, which may be combined, such as tonometry (intraocular pressure), ophthalmoscopy (shape and colour of the optic nerve), perimetry (vision field), gonioscopy (eye angle where the iris meets the cornea), pachymetry (thickness of the cornea) and nerve fibre analysis. Some of the aforementioned tests are complex and they generally require a special and often expensive equipment.

Open-angle glaucoma (OAG) is the one of the most common forms of the disease and it is commonly associated with increased intraocular pressure (IOP), i.e. the tension exerted by the aqueous humor in the intraocular tissues as a result of the balance between its production and drainage, and visual field deterioration. To date, intraocular pressure (IOP) is considered a main prognostic risk factor for OAG.

In-office measurements of IOP are often used to assess diurnal variation of the IOP, yet these measurements may convey only a fraction of a patient's circadian IOP pattern. IOP can vary throughout a 24-hours period due to a number of external factors, such as for example sleep-related lid squeezing/squinting, eye wiping, muscular effort, increased expiratory effort such as during physical exercise or wearing a shirt or tie which are tight around the neck. A cumulative effect of constant or frequent fluctuations of IOP values may lead to misdiagnosis.

Miniaturization of sensor technology has provided new opportunities for IOP monitoring and glaucoma diagnosis.

Patent US 9,968,254 relates to an IOP monitoring device that can be placed on the eye of a user to monitor intraocular pressure over an extended period of time. The device comprises a soft contact lens and a pressure sensor united with said contact lens. The pressure sensor comprises an active strain gage, a passive gage, a rigid element and a microprocessor. The active strain gage, passive gage and rigid element are placed at a distance from the centre of the contact lens. Each of the active and passive strain gage comprises a portion encircling the centre of the contact lens on at least 180°. A kit is also describes, the kit comprising such a pressure monitoring device and a portable recording device configured for communicating with the pressure monitoring device and for storing data received from it.

The IOP monitoring device of US 9,968,254 requires that the antenna and the (bulky) portable power recording device should be continuously worn by the patient for IOP monitoring at the cost of patient's comfort and movement capabilities.

Application EP 2 876 487 A1 describes an energized ophthalmic device and a method of monitoring the IOP of a patient's eye.

WO 2015/095177 A1 concerns an eye mountable device for measuring IOP including a transparent polymeric material having a concave surface and a convex surface, wherein the concave surface is configured to be removably mounted over a corneal surface, an antenna at least partially embedded in the transparent polymeric material to communicate date indicative of the measured resistance deformation of the cornea to an external reader, an expandable member to apply the force to corneal surface, and a sensor to detect a resistance to deformation of the cornea.

Joohee Kim et al. in "Wearable smart sensor systems integrated on soft contact lenses for wireless ocular diagnostics", published in Nature Communications, Vol. 8, 14997 (2017), describe a wearable smart contact lens with highly transparent and stretchable sensors that continuously and wirelessly monitors glucose and intraocular pressure, which are the risk factors associated with diabetes and glaucoma. The multifunctional contact lens sensor measures the glucose level in tear fluid and intraocular pressure simultaneously but yet independently based on different electrical responses.

Patent KR1828511 B1 relates to a contact lens sensor using capacitance change to measure the IOP. The sensing layer is a dielectric layer made of a transparent elastomeric material that changes its thickness in response to a change of IOP. The dielectric layer is arranged between a lower and an upper transparent electrode patterned as a spiral shaped antenna.

Application US 2018/0160976 A1 discloses a contact lens having a biosensor thereon. A transparent, flexible patterned conductor and a transparent, flexible antenna are described comprising a combination of a first electrode formed of a two-dimensional material, such as graphene, and a second electrode made of conductive fibres which can be one-dimensional material, such as nanowires, disposed in a network or mesh on the two dimensional material. The patterned conductor and the antenna are arranged on a contact lens. US 2018/0160976 indicates that the biosensor can be operated by disposing a reader within a predetermined distance of the biosensor, and exciting the antenna with an RF signal. The biosensor is designed to detect a glucose concentration in tears while wearing contact lenses.

EP 2 629 658 A1 discloses a device for measuring IOP including a corneal contact lens having a pressure sensor mounted in a recess or cavity in the contact lens and a responder coil. An apparatus comprising the device is also described in combination with spectacles or an eye mask having an exciter coil which induces an emf in the respondent coil.

WO 03/088867 A1 relates to an apparatus for measuring IOP of an eye comprising a contact lens including an inner surface contoured to a surface portion of the eye for engaging the surface portion, and a sensor disposed in said inner surface of said contact lens. The sensor comprises a contact surface for making contact with the surface portion, the contact surface including an outer non-compliant region and an inner compliant region fabricated as an impedance element that varies in impedance as said inner compliant region changes shape, and a region of conductive material electrically coupled to said impedance element of the compliant region and responsive to an external signal for energizing the impedance element so that the IOP may be determined. The compliant region comprises a diaphragm that functions as one plate of a capacitive element and the region of conductive material comprises an inductor coil that is electrically coupled to the capacitive element to form a resonant circuit.

In EP 2 347 702 A1, an IOP measuring system comprises one or two contact lenses, each contact lens comprising an antenna and a purely passive sensing means, which comprise a capacitor or a piezoelectric transducer. One or two antenna patches are provided external to the one or two contact lenses to achieve an inductive coupling with the circuitry of the contact lens so that signals provided by the sensing means can be received by the antenna patch via the antenna of the contact lens. The antenna patch can be attached to a pair of spectacles.

Patients affected by glaucoma most often require lifelong treatment and follow-up care to preserve vision because of the chronic nature of the disease itself. Therapy of glaucoma aims at reducing the IOP in the eye and thus slowing the progression of visual field deterioration. For example, most forms of OAG are treated with topical ophthalmic solutions, such as eye drops, and occasionally orally administrated agents, which act either on the reduction of aqueous humor production or on the enhancement of the aqueous outflow, or on both. However, factors such as the limited contact time of drug permeation, a lack of manual dexterity or of good vision experienced by the patients, and patient adherence to a self-administered medical treatment may hinder the effectiveness of the medication.

Drug delivery devices have been developed for administering a drug or other active agent into the ocular region. Application US 2010/0239637 describes a drug eluting contact lens comprising a drug release material comprising at least one drug and a hydrogel lens material encapsulating the drug release material. The contact lens provides substantially zero-order release of the at least one drug over a period of time from the first exposure of the lens to the eye or physiologically-suitable saline solution. The release of the drug from the contact lens may be at a rate of at least 0.001 microgram or 1 microgram per hour, for a period of time of at least 120 hours.

Summary

The Applicant has noted that, while a correct and early diagnosis is a relevant issue in the management of the glaucoma disease, an appropriate medical treatment bears a similar importance. Patients with glaucoma most often require lifelong treatment and follow-up care to preserve vision. Daily adherence to the drug prescription is typically recommended to prevent or treat IOP related diseases in healthy and glaucomatous individuals.

The Applicant has realised that a contact lens including a drug-releasing material and provided with a sensor that allows a prolonged and/or regular monitoring of the IOP in a non-invasive manner would provide information on the actual patient's conditions as well as on the effectiveness of the medical treatment. In particular, the capability of monitoring the IOP during the release of a therapeutic drug from the contact lens to the eye may allow an improved clinical management of the patient.

According to an aspect, a contact lens device configured to be worn in an eye of a user is provided. The contact lens device comprises:

a contact lens extending radially about a central axis and comprising a central optical zone and a radially outer zone located concentrically around the central optical zone;

an intraocular pressure sensor arranged on the contact lens, the pressure sensor comprising a sensing layer made of transparent dielectric material, a lower electrode and an upper electrode, the sensing layer being placed between the lower electrode and the upper electrode with respect to the central axis, wherein the pressure sensor is arranged on the central optical zone of the contact lens, the contact lens comprises a drug-releasing material containing a drug, the drug-releasing material being incorporated in the contact lens within the radially outer zone so as to be arranged peripherally the pressure sensor.

Advantageously, a continuous and/or regular feedback of the impact of the medical treatment though non-invasive IOP measurements may be carried out without large discomfort for the patient. A control of patient adherence and compliance with the prescribed therapy may be also facilitated.

Preferably, the contact lens is soft contact lens.

Preferably, the contact lens is a soft contact lens made of a hydrogel material, such as silicone or acrylate hydrogel material.

Preferably, the drug-releasing material is encapsulated by the hydrogel material of the contact lens.

Preferably, the pressure sensor is attached on the contact lens.

Preferably, the contact lens has an upper surface and the pressure sensor is attached on the upper surface of the contact lens.

Preferably, the drug-releasing material extends across a region within the radially outer zone.

Preferably, the drug-releasing material extends across a ring shaped region within the radially outer zone and surrounding the intraocular pressure sensor.

The contact lens has a lens lower surface configured to contact an eye of a wearer and a lens upper surface.

Preferably, the radially outer zone of the contact lens comprises a plurality of micro perforations opening to the lens lower surface. In particular, the plurality of micro perforations are located under the region of the drug-releasing material. Preferably, the plurality of micro perforations extend along a direction substantially parallel to the central axis from the drug-releasing material to the lens lower surface so as to allow discharge of the drug through the micro perforations. For example, micro perforations are through-holes extending from the drug-releasing material to the lens lower surface.

Preferably, the sensing layer, the first electrode and the second electrode extend radially only over the central zone of the contact lens.

Preferably, the lower electrode and the upper electrode of the pressure sensor are made of a transparent conductive material.

Preferably, the sensing layer is made of a transparent elastomeric polymer.

Preferably, the intraocular pressure sensor comprises a base layer arranged on the lens upper surface of the contact lens and a passivation layer positioned above the sensing layer.

Preferably, all layers forming the pressure sensor are made of transparent material.

Preferably, the lower electrode is formed on a surface of the base layer and the upper electrode is formed on a surface of the passivation layer.

Preferably, each of the lower and upper electrodes of the pressure sensor is configured as an inductive coil.

The term "coil" is meant to include single-turn or multiple-turn wound coils, not limited to a particular shape, which may be a helix or spiral.

Preferably, the upper and lower electrodes of the contact lens sensor are configured as coils made of a single conductor spirally wound.

Advantageously, the use of a contact lens sensor according to the present disclosure may allow IOP monitoring and drug-release functionalities to be simultaneously carried out independently from one another.

Within the preferred embodiments according to the present disclosure, by "transparent" is meant a layer or a material having visible light transmittance of at least 90%, preferably from 95% up to 100%.

The present disclosure relates also to a kit comprising contact lens device according to the present disclosure and an external wearable object, wherein the lower and upper electrodes of the contact lens device function as responder coil and the wearable object includes at least one exciter coil connected to a power source for powering the exciter coil.

For example, the wearable object is a pair of spectacles or a night eye mask.

According to the present disclosure, a pressure monitoring system for wirelessly monitoring intraocular pressure is provided, the system comprising:

a contact lens device according to the present disclosure,
an external radio-frequency antenna configured to emit and receive radio-frequency signals, the external radio-frequency antenna acting as an exciter coil creating an inductive communication link with the lower and the upper electrodes of the intraocular pressure sensor, and
a wireless communication device comprising a first radio frequency transceiver in communication with the external radio-frequency antenna and configured to receive radio-frequency signals indicative of the intraocular pressure from the intraocular pressure sensor, a power source operatively connected to the external radio-frequency antenna, and a processing unit configured to receive the radio-frequency signals indicative of the intraocular pressure from the first transceiver, to determine respective intraocular pressure values and to store the determined intraocular pressure values in a memory component operatively connected to the processing unit.

Preferably, the a wireless communication device comprises a second radio-frequency transceiver configured to transmit wireless radio frequency signals, the second radio-frequency transceiver being operatively connected to the memory component and configured to receive the determined intraocular pressure values and to transmit wireless radio frequency signals indicative of the determined intraocular pressure values.

A continuous wireless detection of IOP of a patient is possible for a prolonged time, such as when the patient is at home or sleeping.

Brief description of the drawings

The present disclosure will be now described more fully hereinafter with reference to the accompanying drawings, in which some, but not all embodiments are shown. Drawings illustrating the embodiments are not-to-scale schematic representations.

FIG. 1 is an exploded side view of a contact lens sensor according to an embodiment.
FIG. 1a is an enlarged partial view of the contact lens of the contact lens sensor of Fig. 1 (inset A) shown in cross-sectional view to better highlight some details.
FIG. 2 is a schematic top view of the contact lens sensor of Fig. 1.
FIG. 2a is an enlarged partial top view of Fig. 2 (inset B) to better highlight some details.
FIG. 3 represents diagrammatically a monitoring system for continuous or regular monitoring of the IOP, according to an example.
FIG. 4 is a schematic front view of a pair of spectacles including an exciter coil for energization of the responder coil of the contact lens sensor.
FIG. 5 is a schematic front view of a night eye mask including an exciter coil for energization of the responder coil of the contact lens sensor.

Detailed description

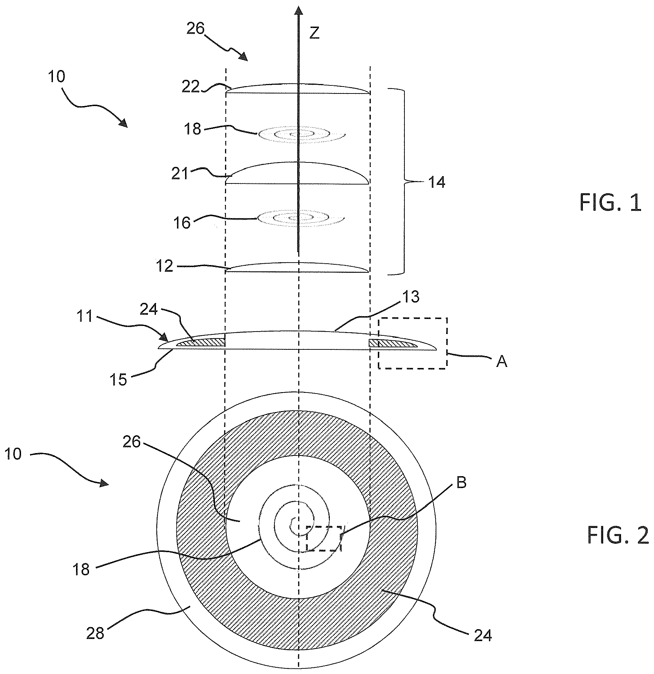

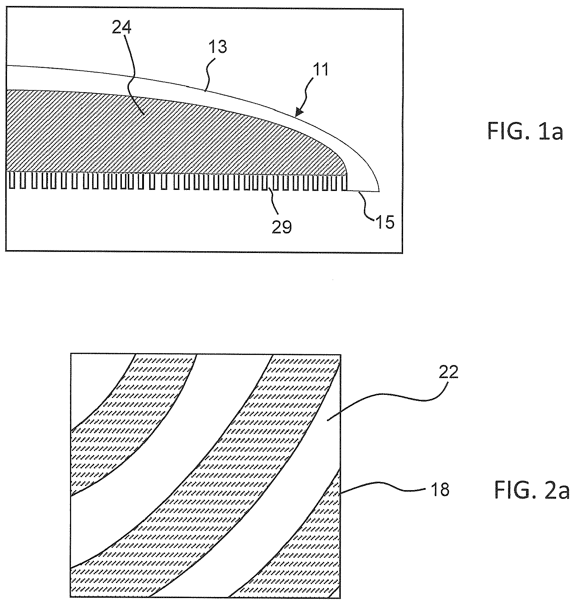

Figure 1 is an exploded side view of the contact lens device according to an embodiment, whereas Fig. 2 is a top view of the contact lens device of Fig. 1. A contact lens device 10 comprises a contact lens 11 extending radially about a central axis Z. The contact lens 11 comprises a central optical zone 26 and a radially outer zone 28 surrounding the central optical zone 26 (Fig. 2). The central optical zone 26 comprises the central axis Z, which corresponds to the axis of symmetry of the contact lens device 10.

Preferably, the contact lens 11 is a soft contact lens. The soft contact lens may be made of a transparent polymeric material, typically a hydrogel material, such as acrylate or silicone hydrogel material.

Typically, the contact lens 11 has a curved shape with an apex at the central axis Z. Typically, the contact lens 11 has a convex lens upper surface 13 and a concave lens lower surface 15 opposite to the lens upper surface (only schematically indicated in the figures). The lens lower surface 15 is to be mounted over a corneal surface of the eye (not shown).

In the present description and claims, "upper", and "lower" are defined with respect to a vertical axis, which corresponds or is parallel to the central axis Z and according to the position of the lens when in use.

The lens upper surface 13 has a central portion corresponding to the central optical zone 26 of the contact lens 11.

The contact lens device 10 comprises an intraocular pressure sensor 14. The pressure sensor 14 is arranged on the contact lens 11 and in particular on the lens upper surface 13. The pressure sensor 14 overlies the central optical zone 26 of the contact lens 11. The pressure sensor 14 comprises a sensing layer 21 made of a dielectric material. The dielectric material of the sensing layer 21 exhibits a dielectric constant. In response to pressure, the dielectric material compresses or more generally changes in thickness thereby causing a dielectric constant variation, which is indicative of the pressure applied to the dielectric material.

The contact lens 11 acts as a substrate for the pressure sensor 14. The pressure sensor 14 is coupled, in particular attached on the contact lens 11. More particularly, the pressure sensor is attached on the lens upper surface 13 of the lens 11, wherein the attachment may comprise bonding and/or lamination between superimposed layers. The pressure sensor may be bonded or otherwise attached to the contact lens.

Preferably, the sensing layer 21 is made of a transparent elastomeric polymer, in particular a pressure-sensitive elastomeric polymer. For example, the pressure-sensitive elastomeric polymer is a silicone elastomer, such as PDMS (polydimethylsiloxane) or ecoflex®.

The sensing layer 21 has an upper convex surface and a lower concave surface.

The pressure sensor 14 comprises a lower electrode 16 and an upper electrode 18. The sensing layer 21 is positioned between the lower electrode 16 and the upper electrode 18 relative to the central axis Z. The lower electrode 16 is placed above the lens upper surface 13 of the contact lens 11.

The lower electrode 16 and the upper electrode 18 are preferably made of a transparent conductive material.

In an example, the lower and upper electrodes 16, 18 are made of a composite material comprising graphene and a mesh of conductive nanofibers or nanowires to form conductive, flexible and transparent elements.

Each electrode may comprise a combination of a two-dimensional material such as, but not limited to, graphene, and conductive fibers which can be made of a one-dimensional material, such as, but not limited to, metal nanowire (mNWs) or their hybrid structures. The conductive fibers may be arranged in a network or mesh on the two-dimensional material with specific electrical and mechanical properties to enhance the functioning of the sensor and, at the same time, to not interfere with vision.

The sensing layer 21 acts as a passive capacitive element that changes capacitance in proportion to the intraocular pressure (IOP) of the eye. Upon deformation of the sensing layer 21, the distance between the two electrodes 16, 18 changes, leading to a change in the capacitance of the capacitor formed by the two electrodes that act as capacitive plates.

Each of the lower and upper electrodes 16, 18 is configured as an inductive coil, in particular as a coil antenna. In the example, electrodes have a spiral shape. As described in the following, the lower and upper electrodes may act as responder coil that can be energized by an external coil when the latter is placed in the proximity of the contact lens device 10.

Figure 2a shows in some more detail an enlarged portion of the upper electrode 18.

The pressure sensor 14 comprises a base layer 12 arranged directly on and overlying the central optical zone 26 the contact lens 11, in particular the central region of the lens upper surface 13 of the contact lens 11. The base layer 12 is interposed between the lens upper surface 13 and the sensing layer 21. The lower electrode 16 is formed on the base layer 12. Preferably, the lower electrode 16 is bonded on a surface of the base layer 12, either the lower surface or the upper surface of the base layer. Preferably, the lower electrode 16 is formed on a surface of the base layer by lithography, such as 3D printing or photolithography.

The pressure sensor 14 comprises a passivation layer 22 covering the underlying sensing layer 21. Preferably, the upper electrode 18 is bonded on a surface of the passivation layer 22, either its upper or lower surface, preferably its lower surface. Preferably, the upper electrode 18 is formed on a surface of the passivation layer by lithography, such as 3D printing or photolithography.

The sensing layer 21 and each of the lower and upper electrodes 16, 18 extend radially over only the central optical zone 26. Preferably, each layer 12, 16, 21, 18, 22 constituting or being included in the pressure sensor 14 radially extends over only the central optical zone 26 of the contact lens 11.

Preferably, the base layer 12 and the passivation layer 22 are made of a transparent polymeric material. Preferably, the base layer and passivation layer are made of a transparent elastomeric polymer. In a preferred example, the base layer and the passivation layer are made of parylene.

Preferably each layer forming the pressure sensor 14 is made of a stretchable and transparent material. Flexibility/stretchability of all polymeric layers forming the pressure sensor allows pressure-sensitive measurements and comfort/wearability of the contact lens device.

In an example, the layers constituting or being included in the pressure sensor 14 are integral to one another, such as by bonding, to form a monolithic structure.

In accordance with the present disclosure, the contact lens 11 includes a drug-releasing material 24 embedded or encapsulated in the contact lens, more generally incorporated, in the contact lens 11. The drug-releasing material is positioned within the radially outer zone 28 of the contact lens 11 so as to be arranged peripherally the pressure sensor 14 and avoid interference with the light incident in the central optical zone 26 of the cornea..

Preferably, the drug-releasing material 24 is encapsulated by the material of the contact lens 11, typically a hydrogel material.

Preferably, the drug-releasing material 24 surrounds the central optical zone 26. In an example, the drug-releasing material 24 extends across an arc-shaped region surrounding the central optical zone 26. In other example, the drug-releasing material has a semi-circular shape. Preferably, the drug-releasing material extends across has a ring shaped region within the radially outer zone 28 to surround the central optical zone 26.

The drug-releasing material 24 contains a therapeutic drug. The drug may be one or more therapeutic agents for glaucoma therapy, such as prostaglandin/prostamides, Rhokinase inhibitors, and/or parasympathomimetics. The drug-releasing material 24 may comprise a delivery polymeric matrix and a drug embedded, encapsulated or otherwise incorporated into the delivery polymeric matrix. For example, the drug can be incorporated in nanoparticles dispersed in the polymer matrix, for example a hydrogel matrix. In an embodiment, the drug-release material includes a polymer, for example poly(lactic-co-glycolic) Acid (PLGA), in which a drug is mixed or dispersed. In an example, the hydrogel matrix of the drug-releasing material and the hydrogel material of the contact lens 11 may be the made of the same material.

The drug contained in the drug-releasing material 24 can diffuse and/or migrate through the contact lens 11 when the contact lens sensor 10 is placed on the eye. Preferably, the contact lens 11 comprises a plurality of micro perforations 29 (visible in Fig. 1a) opening on the lens lower surface 15 of the contact lens 11 to facilitate drug release to the eye. Micro perforations 29 may also provide a sufficient oxygenation of the cornea, which is beneficial in case of a prolonged use of the contact lens sensor 10.

The micro perforations 29 may be vertical through holes extending, in a vertical direction parallel to the central axis Z, from the drug-releasing material 24 to the lens lower surface 15 of the contact lens 11. The micro perforations 29 are located under the drug-releasing material 24 and extend across a radial region corresponding to the region where the drug-releasing material is present.

When monitoring the IOP of the drug-releasing contact lens device according to the present disclosure, a subject is wearing the contact lens device. A wireless communication device comprising an external antenna configured to receive electric RF signals may be placed in the proximity of the contact lens device. The external antenna, typically a coil antenna, is powered so as to receive RF signals from the pressure sensor by creating an inductive communication link between the external antenna acting as an exciter coil and the pressure sensor 14, i.e., electrodes of the pressure sensor 14 of the contact lens device, acting a responder coil.

In order to power the circuit of the capacitor and record the resonant frequency values, a magnetic field is formed between and encompassing a responder coil 16, 18 and an exciter coil to be positioned externally and close to the eye.

As generally known, the operation principle is based on the inductive coupling between the external exciter coil and the inductor-capacitor (LC) circuit of the contact lens device, characterised by a resonant frequency.

The equivalent input impedance $Z_1$ at the output of the exciter coil is

$$Z_1 = R_1 + j2\pi f L_1 \left(1 + \frac{k^2 (f/f_2)^2}{1 + jf/(f_2 Q_2) - (f/f_2)^2}\right) \quad (1)$$

where f is the excitation frequency, k is the fixed coupling coefficient of the inductive link, $Q_2$ is the quality factor and $f_2$ is the resonance frequency of the sensor, respectively.

The value $Z_1$ can be further split up into a real part as:

$$Re(Z_1) = R_1 + 2\pi f L_1 k^2 Q_2 \frac{f/f_2}{1 + Q_2^2 (f/f_2 - f_2/f)^2} \quad (2)$$

If $Q_2 \gg 1$, $Re(Z_1)$ reaches the maximum value when $f = f_2$. Equation (2) shows that the measured resonance frequency of the pressure sensor is independent from the reading distance and reading angle between the read-out coil and the pressure sensor when $Q_2 \gg 1$. Consequently, the resonance frequency of the sensor can be measured based on the real part of the input impedance of the reading coil $Re(Z_1)$.

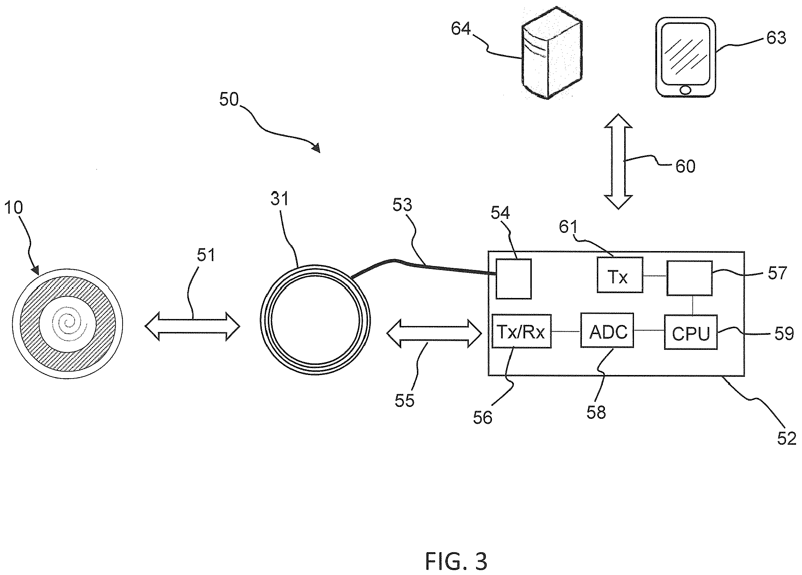

Figure 3 is a diagram of a monitoring system 50 for continuous or regular monitoring of the IOP according to an example. An external coil 31 acting as an external antenna, mounted on a wearable object (not shown), is placed at the proximity of the contact lens device 10. The exciter coil 31 is operatively connected with a power source 54, such a battery, for powering the exciter coil and generate a magnetic field that interacts with the responder coil. An inductive link 51 created by the magnetic field, which typically is a radio frequency (RF) link, energizes the LC passive circuit of the contact lens device 10.

In the example of Fig. 3, the monitoring system 50 comprises a wireless communication device 52 comprising the power source 54 electrically connected, for example by means of cable 53, to the external coil 31. The wireless communication device 52 may be a portable or wearable device. Once energized, the pressure sensor 14 of the contact lens sensor 10 transmits RF signals indicative of the instantaneous IOP to a first RF transceiver 56 in the communication device 52 (wireless communication link 55). The communication device 52 comprises an analog-to-digital converter (ADC) 58 operatively connected to the first RF transceiver 56 and an electronic processing unit 59, such as a microcontroller, operatively connected to the ADC 58. The ADC 58 is designed to receive RF analog electrical signals from the first RF transceiver 56 and convert them into RF digital signals, possibly after amplification of the received signals (not shown). The processing unit 59 is configured to receive the digital signals indicative of the instantaneous IOP and determine IOP values in the digital signals, representing the measured instantaneous IOP values. The processing unit 59 is configured to store the determined IOP values in a memory component 57 operatively connected to the processing unit 59.

In general, the electronic processing unit 59 is configured to process and control operations and functions of the wireless communication device 52 device by executing software code. The processing unit 59 may be implemented using one or more separate central processing units (CPUs), application specific integrated circuits (ASICs), digital signal processing devices (DSPDs), programmable logic devices (PLDs), or field programmable gate arrays (FPGAs). The memory component 57 may be a semiconductor memory device, such as Synchronous Dynamic Random Access Memory (SDRAM), Random Access Memory (RAM) device, a static RAM (SRAM), a Read Only Memory (ROM) device, an electrically erasable programmable ROM (EEPROM) or a programmable ROM (PROM).

The wireless communication device 52 comprises a second wireless transceiver 61, typically a short- or medium-range wireless RF transceiver, configured to transmit wireless RF signals. Short or medium range wireless communication may be performed, for example, using Wi-Fi, Bluetooth or ZigBee.

Although not indicated in Fig. 3, the power source 54 powers all electronic components of the communication device 52.

In particular, data indicative of the instantaneous IOP determined by the processing unit 59 are stored in the memory component 57 and transmitted to the second RF transceiver 61 that, depending on the wireless technology used, transmit them to a remote computer or a wireless device via wireless communication link 60. For example, IOP indicative data are transmitted to a remote server 64 or to a mobile phone or tablet 63 in wireless connection with the wireless communication device 52.

Data indicative of the instantaneous IOP may be remotely monitored, for example by processing the data into an intraocular pressure curve to be stored and analysed by medical staff. In an example, an alarm may be triggered when the IOP values are determined to be above an IOP threshold value.

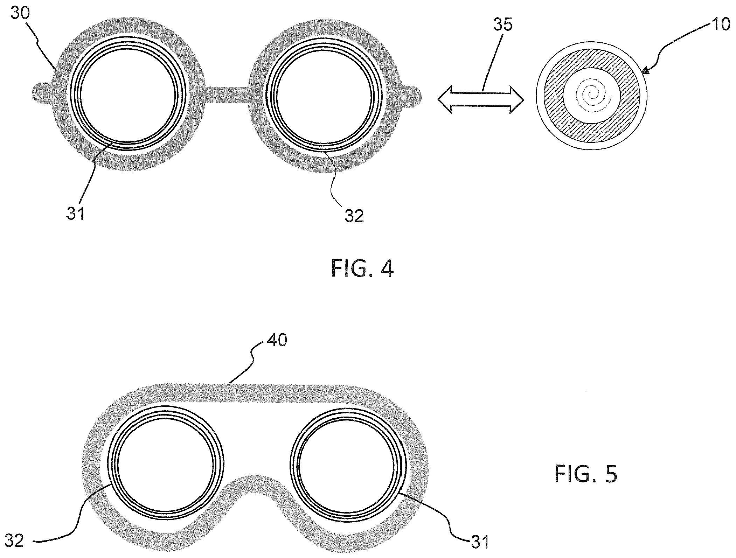

Figure 4 schematically illustrates a kit for IOP monitoring according to an embodiment. The kit comprises a contact lens device 10, which can be of the type described with reference to Figs. 1 and 2, and an external wearable object 30 that can be worn by a subject wearing the contact lens sensor 10. In the example, the wearable object 30 is a pair of spectacles. The pair of spectacles comprises at least one external antenna 31, in particular a first and a second external antennas 31, 32, assuming that the subject is wearing a respective contact lens device in both eyes.

When worn by the subject, the at least one external antenna 31, 32 is positioned sufficiently close to the responder coil in the contact lens sensor 10 to act as exciter coil and create an inductive communication link 36 with the respondent coil 16, 18 of the pressure sensor 14. Each exciter coil 31, 32 is preferably placed coaxially with respect to a contact lens device 10 and in particular with the electrodes 16, 18 functioning as responder coil.

The at least one external antenna 31, 32 is made of a conductive material and may be configured with a spiral or helical shape. For example, each exciter coil may be shaped as a single turn or multi turn spiral-shaped antenna. The external antenna 31, 32 may be made of a thin, transparent and conductive material, typically indium tin oxide (ITO).

One or two exciter coils may be mounted on the spectacle's frame or on a peripheral region of each lens of the spectacles so as not to interfere with the subject's vision. The exciter coil is operatively connected with a power source (not shown), such a battery, for powering the exciter coil and generate a magnetic field.

Figure 5 shows a further example of wearable object to be placed in the proximity of the contact lens sensor for IOP monitoring. Exciter coils 31, 32 are placed on an outer surface of a night eye mask 40.

Those skilled in the art will recognize that it is possible to combine the various features of the embodiments described above in order to obtain further embodiments, all of which are in any case encompassed by the present disclosure as defined by the following claims.

**Claims**

1. A contact lens device (10) comprising:

   a contact lens (11) extending radially about a central axis (Z) and comprising a central optical zone (26) and a radially outer zone (28) located concentrically around the central optical zone (26);
   an intraocular pressure sensor (14) arranged on the contact lens (11), the pressure sensor (10) comprising a sensing layer (21) made of transparent dielectric material, a lower electrode (16) and an upper electrode (18), the sensing layer (21) being placed between the lower electrode (16) and the upper electrode (18) with respect to the central axis (Z), wherein
   the pressure sensor (14) is arranged on the central optical zone (26) of the contact lens (11), the contact lens (11) comprises a drug-releasing material (24) containing a drug, the drug-releasing material (24) being incorporated in the contact lens (11) within the radially outer zone (28) so as to be arranged peripherally the pressure sensor (14).

2. The contact lens device (10) of claim 1, wherein the contact lens (11) is a soft contact lens made of a hydrogel material.

3. The contact lens device of claim 2, wherein the drug-releasing material (24) is encapsulated by the hydrogel material of the contact lens (11).

4. The contact lens device of claim 2 or 3, wherein the drug-releasing material (24) extends across a ring shaped region within the radially outer zone (28) and surrounding the intraocular pressure sensor (14).

5. The contact lens device (10) of any one of the preceding claims, wherein the contact lens has a lens lower surface (15) configured to contact an eye of a wearer, the drug-releasing material (24) extends across a region within the radially outer zone (28) of the contact lens (11) and the contact lens (11) comprises a plurality of micro perforations (29) located under the region of the drug-releasing material and extending in a direction substantially parallel to the central axis from the drug-releasing material to the lens lower surface (15) so as to allow discharge of the drug through the micro perforations (29).

6. The contact lens device (10) of any one of the preceding claims, wherein the sensing layer (21), the first electrode (16) and the second electrode (18) extend radially only over the central zone (26) of the contact lens (11).

7. The contact device (10) of any one of the preceding claims, wherein the lower electrode (16) and the upper electrode (18) are made of a transparent conductive material and wherein the sensing layer (21) is made of a pressure-sensitive elastomeric polymer.

8. The contact lens device (10) of any one of the preceding claims, wherein the contact lens has a lens upper surface (13) and the intraocular pressure sensor (14) comprises a base layer (12) arranged on the lens upper surface (13) of the contact lens (11) and a passivation layer (22) located above the sensing layer (21), and wherein the lower electrode (16) is formed on a surface of the base layer (12) and the upper electrode (18) is formed on a surface of the passivation layer (22).

9. The contact device (10) of any one of the preceding claims, wherein each of the lower and upper electrodes (16, 18) is configured as an inductive coil.

10. A kit comprising the contact lens device (10) according to claim 9 and an external wearable object (30; 40), wherein the lower and upper electrodes (16, 18) of the contact lens device (10) function as responder coil and the wearable object (30; 40) includes at least one exciter coil (31, 32) connected to a power source (54) for powering the exciter coil (31, 32).

11. A monitoring system (50) for wirelessly monitoring intraocular pressure comprising:

a contact lens device (10) according to one or more of claims 1 to 9,
an external radio-frequency antenna (31, 32) configured to emit and receive radio-frequency signals, the external radio-frequency antenna (31, 32) acting as an exciter coil creating an inductive communication link with the lower and the upper electrodes (16, 18) of the intraocular pressure sensor (14), and
a wireless communication device (52) comprising a first radio frequency transceiver (56) in communication with the external radio-frequency antenna (31, 32) and configured to receive radio-frequency signals indicative of the intraocular pressure from the intraocular pressure sensor (14), a power source (54) operatively connected to the external radio-frequency antenna (31, 32), and a processing unit (59) configured to receive the radio-frequency signals indicative of the intraocular pressure from the first transceiver (56), to determine respective intraocular pressure values and to store the determined intraocular pressure values in a memory component (57) operatively connected to the processing unit (59).

12. The monitoring system (50) of claim 11, wherein the a wireless communication device (52) comprises a second radio-frequency transceiver (61) configured to transmit wireless radio frequency signals, the second radio-frequency transceiver (61) being operatively connected to the memory component (57) and configured to receive the determined intraocular pressure values and to transmit wireless radio frequency signals indicative of the determined intraocular pressure values.

FIG. 1

FIG. 2

FIG. 1a

FIG. 2a

EP 3 861 924 A1

FIG. 3

FIG. 4

FIG. 5

EP 3 861 924 A1

Europäisches
Patentamt

European
Patent Office

Office européen
des brevets

# EUROPEAN SEARCH REPORT

Application Number

EP 21 15 4672

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/380871 A1 (GUTIERREZ CHRISTIAN [US]) 19 December 2019 (2019-12-19) * paragraphs [0032], [0036], [0037], [0040], [0041], [0049] * ----- | 1-12 | INV. A61B3/16 A61F9/00 |
| A | WO 2009/100439 A2 (EHRECKE TIMOTHY J [US]) 13 August 2009 (2009-08-13) * page 5, lines 5-20 * * page 8, lines 8-15 * * page 9, lines 15-20 * ----- | 1-12 | |
| Y,D | KR 2017 0139300 A (UNIST(ULSAN NAT INSTITUTE OF SCIENCE AND TECHNOLOGY) [KR]) 19 December 2017 (2017-12-19) * paragraphs [0006] - [0008], [0016], [0020] * ----- | 1-12 | |

TECHNICAL FIELDS
SEARCHED (IPC)

A61B
A61F
G02C

The present search report has been drawn up for all claims

| Place of search | Date of completion of the search | Examiner |
|---|---|---|
| Munich | 14 June 2021 | Martelli, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 15 4672

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2021

| Patent document cited in search report | | Publication date | Patent family member(s) | | Publication date |
|---|---|---|---|---|---|
| US 2019380871 | A1 | 19-12-2019 | EP US WO | 3809949 A1 2019380871 A1 2019246216 A1 | 28-04-2021 19-12-2019 26-12-2019 |
| WO 2009100439 | A2 | 13-08-2009 | US WO | 2009203985 A1 2009100439 A2 | 13-08-2009 13-08-2009 |
| KR 20170139300 | A | 19-12-2017 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9968254 B
- EP 2876487 A1
- WO 2015095177 A1
- KR 1828511 B1
- US 20180160976 A1
- US 20180160976 A
- EP 2629658 A1
- WO 03088867 A1
- EP 2347702 A1
- US 20100239637 A

**Non-patent literature cited in the description**

- **JOOHEE KIM et al.** Wearable smart sensor systems integrated on soft contact lenses for wireless ocular diagnostics. *Nature Communications,* 2017, vol. 8, 14997